(19) Europäisches Patentamt
European Patent Office
Office européen des brevets

(11) EP 0 513 335 B1

(12) EUROPEAN PATENT SPECIFICATION

(45) Date of publication and mention
of the grant of the patent:
**26.03.1997 Bulletin 1997/13**

(21) Application number: 92902214.3

(22) Date of filing: 04.12.1991

(51) Int. Cl.⁶: **A61B 17/06**, D02G 3/02

(86) International application number:
**PCT/US91/09137**

(87) International publication number:
**WO 92/10137 (25.06.1992 Gazette 1992/14)**

(54) **CABLED CORE AND BRAIDED SUTURE MADE THEREFROM**

SEELE AUS DRÄHTEN SOWIE DARAUS GEFLOCHTENES NAHTMATERIAL

AME CABLEE ET FIL TRESSE POUR SUTURES POSSEDANT UNE TELLE AME

(84) Designated Contracting States:
**BE CH DE ES FR GB IT LI LU NL SE**

(30) Priority: **05.12.1990 US 623224**
**19.07.1991 US 733362**

(43) Date of publication of application:
**19.11.1992 Bulletin 1992/47**

(73) Proprietor: **United States Surgical Corporation**
**Norwalk, Connecticut 06856 (US)**

(72) Inventors:
 • **CHESTERFIELD, Michael, P.**
 **Norwalk, CT 06851 (US)**
 • **KOYFMAN, Ilya**
 **Orange, CT 06477 (US)**
 • **KAPLAN, Donald, S.**
 **Weston, CT 06612 (US)**

 • **HERMES, Matthew, E.**
 **Easton, CT 06612 (US)**

(74) Representative: **Marsh, Roy David et al**
 **Hoffmann Eitle & Partner**
 **Patent- und Rechtsanwälte**
 **Postfach 81 04 20**
 **81904 München (DE)**

(56) References cited:
**DE-A- 2 949 920       US-A- 3 125 095**
**US-A- 3 187 752       US-A- 3 359 983**
**US-A- 3 443 451       US-A- 3 780 515**
**US-A- 4 047 533       US-A- 4 321 038**
**US-A- 4 351 146       US-A- 4 414 800**
**US-A- 4 484 436       US-A- 4 546 769**
**US-A- 4 712 553       US-A- 4 946 467**
**US-A- 4 959 069       US-A- 4 974 408**
**US-A- 5 012 636       US-A- 5 019 093**

**Description**

BACKGROUND OF THE INVENTION

This invention relates to a braided suture of improved construction featuring a cabled core component.

Sutures intended for the repair of body tissues must meet certain requirements: they must be substantially non-toxic, capable of being readily sterilized, they must have good tensile strength and have acceptable knot-tying and knot-holding characteristics and if the sutures are of the absorbable or biodegradable variety, the absorption or biodegradation of the suture must be closely controlled.

Sutures have been constructed from a wide variety of materials including surgical gut, silk, cotton, a polyolefin such as polypropylene, polyamide, polyglycolic acid, polyesters such as polyethylene terephthalate and glycolide-lactide copolymer, etc. Although the optimum structure of a suture is that of a monofilament, since certain materials of construction would provide a stiff monofilament suture lacking acceptable knot-tying and knot-holding properties, sutures manufactured from such materials are preferably provided as braided structures. Thus, for example, sutures manufactured from silk, polyamide, polyester and bioabsorbable glycolide-lactide are braided to provide optimum knot-tying and knot-holding properties.

Currently available braided suture products are acceptable in terms of their knot-tying and knot-holding properties. However, as removed from the package, they tend to be stiff and wiry and retain a "set" or "memory" such that at the time of use, it is usually necessary for the surgeon or assistant personnel to flex and stretch the suture to make it more readily handleable. Furthermore, the surfaces of known sutures are perceptibly rough. Thus, if one passes one's hand or fingers along the braid, surface irregularities will be readily detected. The result of this rough surface is that the suture will exhibit drag or chatter as it is drawn through tissue, characteristics which militate against smooth, neat, accurately placed wound approximation so necessary to excellence in surgical practice.

By way of overcoming the foregoing disadvantages which characterize commercially available braided sutures, U.S. Patent No. 5,019,093 describes a braided suture of improved construction possessing a significantly greater number of sheath yarns for a given overall denier, the sheath yarns being fabricated from individual filaments of finer denier than filaments which are typical of known types of braided suture, the resulting improved suture exhibiting perceptibly improved flexibility and handling and reduced chatter and drag compared with braided sutures of known construction.

In all but the smallest size, the sutures of U.S. Patent No. 5,019,093 can optionally be constructed around a filamentous core, a particularly advantageous feature in sutures of heavier denier. In some of these core-containing suture constructions, the larger number of sheath yarns in the braid may require a larger diameter core relative to that of a core of a similar braided suture constructed from fewer sheath yarns. However, a difficulty has been observed during the normal processing and handling of sutures possessing a somewhat larger core, namely, the core has a tendency in some cases to work through the surface sheath yarns. This "core popping" tendency can be diminished or eliminated by increasing the braid angle (the angle at which sheath yarns cross over each other in the braid). However, this solution to the problem of core popping can only come at the expense of surface smoothness of the suture since such smoothness decreases as the braid angle increases.

In claim 1 below, the pre-characterising portion corresponds to the disclosure of US-A-4946467.

SUMMARY OF THE INVENTION

The present invention aims to provide such a suture possessing a greater number of sheath yarns, a finer denier for the individual filaments contained in an individual sheath yarn and a greater pick count (crossovers per linear inch) for a suture of any given overall denier while at the same time resisting any tendency of its core to penetrate through the outer surface of the suture.

In keeping with these and other objects of the invention, there is provided a braided suture in accordance with claim 1 below.

The cabled core in accordance with the invention provides a balanced structure which remains straight and does not have a tendency to twist upon itself or protrude through the sheath. Individual yarns are twisted in a first direction, the "front" direction, and then multiple twisted yarns are twisted together in the reverse, "back" direction to form a cabled core. The cabled core has a coefficient of twist, alpha, of at least about 24. It is also contemplated that multiple yarns may be plied together, i.e., aligned parallel to one another, with the plied yarns being front twisted and multiple plied yarns being back twisted to form the cabled core of the invention.

The term "suture" as used herein is intended to embrace both non-absorbable as well as the bioabsorbable varieties.

The term "braid" or "braided" as applied to the suture of this invention refers to an arrangement of discrete units, or bundles, denominated "sheath yarns", made up of individual filaments with individual sheath yarns interlocking or interlacing each other in a regular criss-cross pattern.

The term "cabled" as applied to the core component of the braided suture of this invention refers to a core made up of individual yarns each of which has been given a twist in one direction, the twisted yarns being combined to form a core which is then given a twist in a second, opposite direction.

The term "pick count" as applied to a braided suture construction refers to the number of crossovers of sheath yarns per linear inch of suture and, together with the overall denier of the suture, the denier of the individual filaments constituting a sheath yarn and the number of sheath yarns employed, defines the principal construction characteristics of a braided suture.

DESCRIPTION OF THE PREFERRED EMBODIMENTS

In a preferred embodiment, the braided suture of the present invention is fabricated from a bioabsorbable or biodegradable resin such as one derived from polyglycolic acid, glycolide, lactic acid, lactide, dioxanone, trimethylene carbonate, etc., and various combinations of these and related monomers. Sutures prepared from resins of this type are known in the art, e.g., as disclosed in U.S. Patent Nos. 2,668,162; 2,703,316; 2,758,987; 3,225,766; 3,297,033; 3,422,181; 3,531,561; 3,565,077; 3,565,869; 3,620,218; 3,626,948; 3,636,956; 3,736,646; 3,772,420; 3,773,919; 3,792,010; 3,797,499; 3,839,297; 3,867,190; 3,878,284; 3,982,543; 4,047,533; 4,060,089; 4,137,921; 4,157,437; 4,234,775; 4,237,920; 4,300,565; and, 4,523,591; U.K. Patent No. 779,291; D.K. Gilding et al., "Biodegradable polymers for use in surgery -- polyglycolic/poly(lactic acid) homo and co-polymers: 1, Polymer, Volume 20, pages 1459-1464 (1979), and D.F. Williams (ed.), Biocompatibility of Clinical Implant Materials, Vol. II, ch. 9: "Biodegradable Polymers" (1981).

Although the advantages of a cabled core in accordance with this invention, in particular, the resistance to "core popping" mentioned above, are conferred upon braided sutures generally which have been constructed with such a core, they are most fully realized in a braided suture constructed in accordance with the principles established in U.S. Patent No. 5,019,093 referred to above. The defining characteristics of the braided suture of U.S. Patent No. 5,019,093, apart from the material of its construction, are:

(1) overall suture denier;
(2) the pattern of the interlocking yarns expressed in pick count, which is to say, the number of crossovers of individual sheath yarns per linear inch of suture;
(3) the number of sheath yarns comprising the braid;
(4) the denier of the individual filaments comprising each sheath yarn; and,
(5) the denier of the core.

(1) Overall Denier of the Suture

The overall denier of the preferred braided suture can vary from about 125 to about 4000. Within this range, the ranges of overall denier for particular sutures are: from above about 125 to about 200 denier; from above about 200 to about 300 denier; from above about 300 to about 500 denier; from above about 500 to about 800 denier; from above about 800 to about 1200 denier; from above about 1200 to about 2000 denier; and, from above about 2000 to about 4000 denier.

(2) Pattern of the Interlocking Sheath Yarns (Pick Count)

For a preferred suture of any range of overall denier, pick count can vary from about 20 to about 40 crossovers/cm (about 50 to about 100 crossovers/inch) with about 22 to about 32 crossovers/cm (about 55 - 80 crossovers/inch) being preferred. For preferred sutures constructed within any range of overall denier, as larger numbers of sheath yarns are employed, the pick-count for acceptable sutures will also increase within the above ranges. For a preferred suture of a particular range of denier and number of sheath yarns, pick count is advantageously established to achieve a balance in the properties desired. For preferred sutures of any specific denier range and number of sheath yarns, it is preferable to have as low a pick count as possible in order to achieve optimum surface smoothness.

(3) The Number of Sheath Yarns

In the preferred suture, the number of sheath yarns bears some relation to overall suture denier, the number generally increasing with the weight of the suture. Thus, across the range of suture weight (denier) indicated above, the preferred braided suture can be constructed with from about 4 up to as many as about 36 individual sheath yarns constructed from individual filaments having the deniers discussed below.

Table I below sets forth broad and preferred ranges for the numbers of sheath yarns which are suitable for the construction of preferred braided sutures of various ranges of overall denier. The pick counts of the preferred sutures vary from about 50 to about 100 and deniers of individual filaments vary from about 0.2 to about 6.0 for the broad range of number of sheath yarns and the pick counts vary from about 55 to about 80 and the deniers of individual filaments vary from about 0.8 to about 3.0, and advantageously from about 1.0 to about 1.8, for the preferred range of number of sheath yarns:

Table I

| Number of Sheath Yarns Related to Suture Denier | | | |
|---|---|---|---|
| Overall Suture Denier | Suture Size | Number of Sheath Yarns (Broad Range) | Number of Sheath Yarns (Preferred Range) |
| greater than about 125 to about 200 | 6/0 | 4-16 | 6-14 |
| greater than about 200 to about 300 | 5/0 | 4-16 | 6-14 |
| greater than about 300 to about 500 | 4/0 | 10-20 | 12-14 |
| greater than about 500 to about 800 | 3/0 | 14-20 | 14-18 |
| greater than about 800 to about 1200 | 2/0 | 16-32 | 20-30 |
| greater than about 1200 to about 2000 | 0 | 20-36 | 24-32 |
| greater than about 2000 to about 4000 | 1,2 | 20-36 | 24-32 |

While the sheath yarns need not be twisted, it is generally preferred that they be provided with a twist so as to minimize snagging during braid construction. Alternatively, the sheath yarns can be air entangled.

(4) Individual Filament Denier

In the preferred sutures, the individual filaments comprising each sheath yarn can vary in weight. For smaller sutures, i.e. sutures having an overall suture denier of less than about 300, the individual sheath filaments can vary in weight from about 0.2 to about 3.0 denier, preferably from about 1.0 to about 1.8 denier. For larger sutures, i.e. sutures having an overall suture denier of greater than about 300, individual sheath filaments can vary in weight from about 0.2 to about 6.0 denier, preferably from about 0.8 to about 3.0 denier and more preferably from about 1.0 to about 1.8 denier. The number of such filaments present in a particular sheath yarn will depend on the overall denier of the suture as well as the number of sheath yarns utilized in the construction of the suture. Table II sets forth some typical numbers of filaments per sheath yarn for both the broad and preferred ranges of filament weight:

Table II

| Number of Filaments per Sheath Yarn | | |
|---|---|---|
| Approximate Minimum | Approximate Maximum | Filament Denier |
| 45 | 450 | 0.2 |
| 15 | 150 | 0.5 |
| 5 | 50 | 1.5 |
| 3 | 40 | 1.8 |
| 1 | 15 | 6.0 |

The individual filaments of the braided suture may be fabricated from a bioabsorbable polymer derived at least in part from one or more monomers selected from the group consisting of glycolic acid, glycolide, lactic acid, and lactide. Alternatively, the individual filaments may be fabricated from a non-absorbable material, e.g., cotton, silk, polyamide or polyolefin.

(5) Cabled Core

Whether or not provided as a component of the preferred type of braided suture construction described above or some other type of braided suture construction, the cabled core component of the braided suture herein is manufactured in a separate operation and is assembled from a plurality of individual yarns, e.g., from about 2 to about 1500, and preferably from 3 to about 1323 yarns. Each yarn comprising the core is given a twist in one direction, the "front" direction, the twisted yarns then being combined into a core which is then twisted in the opposite direction, the "back" direction, to provide the cabled core unit around which the remainder of the suture is constructed. Depending upon the material used to construct the core, it may be desirable to heat set and/or stretch the core in a known manner prior to final assembly of a braided suture incorporating the cabled core.

The denier of the individual yarns comprising the core is not particularly critical and can range in most cases from about 10 to about 100 and preferably from about 20 to about 70. The degree of twist which is applied to the individual yarns can vary widely with from about 200 to about 1500 turns per meter, and preferably, from about 240 to about 1200 turns per meter, generally providing good results.

The overall denier of the cabled core is, of course, determined by the number and individual deniers of the core yarns from which the core is constructed. For many suture constructions, core denier will range from about 20 to about 80 and preferably from about 25 to about 50 in the smallest size suture and from about 800 to about 2400 and preferably from about 1000 to about 2200 in the largest size suture. In order to increase the total core denier, it is contemplated that for larger suture cores it may be desirable to ply two or more yarns together, preferably before front twisting of the yarns.

The degree of reverse-direction or "back" twist should be determined for a particular suture construction so as to provide a balanced structure. Yarn-to-yarn twist levels can be compared through the "coefficient of twist function" alpha which is related to the helical angle. The coefficient of twist function can be calculated from the relationship

$$\text{alpha} = \frac{K}{100} \sqrt{0.111D} \tag{1}$$

in which alpha is the coefficient of twist, K is the twist level in turns per meter and D is the denier of the resulting twisted product, e.g., twisted yarn or core. In other words, the coefficient of twist, alpha, is given by the product of yarn twist, K, and the square root of yarn count, i.e. mass per unit length or linear density. This coefficient of twist is also directly proportional to the helical or twist angle in yarns having the same solid density (or melt density), so alpha determined for one material can be applied to materials of the same density. Therefore, yarns having different deniers and the same coefficient of twist are geometrically similar, with the coefficient of twist thus being independent of the yarn denier.

The coefficient of twist in the cabled core construction is at least about 24, preferably at least about 28 and most preferably at least about 32.

To apply equal deformation to yarns of different denier, the coefficient of twist of the yarns should be the same. Yarns with higher deformation will have a higher coefficient of twist. For many braided suture constructions, the twist level in turns per meter of the cabled core component can range from about 200 to about 1500 turns per meter and preferably from about 240 to about 1200 turns per meter.

More specifically, the cabled core is constructed from a plurality of individual yarns possessing a first twist of from about 100 to about 1500 turns per meter, the twisted yarns being assembled into a core possessing a second, opposite twist of from about 100 to about 1200 turns per meter. Preferably, the individual yarns possess a first twist of from about 200 to about 1200 turns per meter, with the second opposite twist of the core being about 200 to about 1100 turns per meter.

In practice, once the desired overall core denier and denier of the yarns used to make the core are known, the coefficient of twist may be calculated for the overall core and individual yarns in order to approximate a balanced core structure. Equation (1) is first used to determine the degree of back twist, K, required to obtain a core of the desired overall core denier having a desired coefficient of twist, alpha, greater than 24. Using the same value for the coefficient of twist, equation (1) is then solved using the denier of the individual yarns to determine the approximate degree of front twist which must be applied to the individual yarns in order to obtain a balanced structure.

If multiple plied yarns are to be back-twisted to form the cabled core, the plied yarns are front twisted using equation (1) to determine the degree of twist required to obtain a balanced structure. After the cabled core is constructed, a simple loop test can be performed to determine whether a balanced structure has been achieved. In a common test used by applicants, a one meter length of core is sampled and the ends of the core sample are brought together with the remainder of the core allowed to hang and form a loop. If the loop remains substantially open, i.e., without twisting or with a twist less than 360°, the core structure is considered balanced. However, if the loop twists more than 360° (2 turns) in either direction, the structure is unbalanced and should be modified accordingly. It should be noted that if a cabled core has too much twist in one direction, then the loop will take a twist in the opposite direction. Thus, if the loop twists in a back direction (z), the amount of twist applied to the core or yarn in that back direction (z) should be increased, or the degree of twist in the opposite front direction(s) should be decreased. As will be appreciated, minor variations may be required in order to obtain the desired balanced core structure.

For the preferred suture constructions of U.S. Patent No. 5,019,093, applicants have found that suture size 7/0 and smaller can be made without a core component, and that a core optionally can be included in sutures of size 6/0. Applicants have further found that cores for suture sizes 4/0, 5/0 and 6/0 can be constructed by twisting individual yarns in the front direction and then reverse-twisting individual yarns together to form the cabled core of the invention. For sutures of size 3/0 and larger, it is preferred to obtain the desired overall core denier by plying multiple yarns together, then front twisting and then back twisting the multiple plied yarns together to form the cabled core. In describing the cabled core, it is useful to identify the number of filaments in the structure in terms of the number of filaments in each yarn times the number of yarns plied together times the number of plied yarns which are back twisted together to form the cabled core. This terminology for the number of filaments in the core can be summarized in formula (2) as follows:

$$
\underbrace{\underbrace{60}_{\substack{\text{Number of} \\ \text{filaments} \\ \text{per yarn}}} \times 7}_{\substack{\text{Number of filaments in} \\ \text{each set of plied yarns}}} \times 3
$$

$$\underbrace{\phantom{\underbrace{\underbrace{60}_{\text{filaments}} \times 7}_{\text{plied yarns}} \times 3}}_{\text{Number of filaments in the core}} \tag{2}$$

It will be understood that in the foregoing example, three sets of plied yarns are back twisted to form the core, with each front twisted plied yarn consisting of seven 60 filament yarns.

The following tables and examples set forth describe core and suture constructions suitable for making sutures in accordance with U.S. Patent No. 5,019,093. It will be understood that the core construction may be varied within the scope of the invention for use in constructing sutures outside the scope of the foregoing patent.

Table III below provides some typical core deniers for sutures of various deniers:

Table III

| Core Denier Related to Suture Denier | | | |
|---|---|---|---|
| Overall Suture Denier | Suture Size | Denier of Cabled Core (Broad Range) | Denier of Cabled Core (Preferred Range) |
| greater than about 125 to about 200 | 6/0 | 20-80 | 25-50 |
| greater than about 200 to about 300 | 5/0 | 30-100 | 50-80 |
| greater than about 300 to about 500 | 4/0 | 80-150 | 80-120 |
| greater than about 500 to about 800 | 3/0 | 150-300 | 180-280 |
| greater than about 800 to about 1200 | 2/0 | 250-700 | 350-650 |
| greater than about 1200 to about 2000 | 0 | 400-1200 | 500-1000 |
| greater than about 2000 to about 4000 | 1,2 | 800-2400 | 1000-2200 |

Based on the foregoing, for a given preferred suture construction of a particular overall suture denier, the range of pick count, number of sheath yarns, denier of individual filaments and denier of the cabled core are related to each other as follows:

Table IV

| Braided Suture Construction Parameters | | | | |
|---|---|---|---|---|
| Overall Suture Denier | Pick Count | Number of Sheath Yarns | Denier of Individual Filaments | Denier of Cabled Core |
| greater than about 125 to about 200 | from about 50 to about 100 | from about 4 to about 16 | from about 0.2 to about 1.8 | 20-80 |
| greater than about 200 to about 300 | from about 50 to about 100 | from about 4 to about 16 | from about 0.2 to about 1.8 | 30-100 |
| greater than about 300 to about 500 | from about 50 to about 100 | from about 10 to about 20 | from about 0.2 to about 6.0 | 80-150 |
| greater than about 500 to about 800 | from about 50 to about 100 | from about 14 to about 20 | from about 0.2 to about 6.0 | 150-300 |
| greater than about 800 to about 1200 | from about 50 to about 100 | from about 16 to about 32 | from about 0.2 to about 6.0 | 250-700 |
| greater than about 1200 to about 2000 | from about 50 to about 100 | from about 20 to about 36 | from about 0.2 to about 6.0 | 400-1200 |
| greater than about 2000 to about 4000 | from about 50 to about 100 | from about 20 to about 36 | from about 0.2 to about 6.0 | 800-2400 |

In yet a more preferred braided suture construction, the relationship of the structural elements of the suture for a given suture size is set forth in Table V as follows:

Table V

| More Preferred Braided Suture Construction Parameters | | | | |
|---|---|---|---|---|
| Overall Suture Denier | Pick Count | Number of Sheath Yarns | Denier of Individual Filaments | Denier of Cabled Core |
| greater than about 125 to about 200 | from about 55 to about 80 | from about 6 to about 14 | from about 0.8 to about 1.8 | 25-50 |
| greater than about 200 to about 300 | from about 55 to about 80 | from about 6 to about 14 | from about 0.8 to about 1.8 | 50-80 |
| greater than about 300 to about 500 | from about 55 to about 80 | from about 12 to about 14 | from about 0.8 to about 3.0 | 80-120 |
| greater than about 500 to about 800 | from about 55 to about 80 | from about 14 to about 18 | from about 0.8 to about 3.0 | 180-280 |
| greater than about 800 to about 1200 | from about 55 to about 80 | from about 20 to about 30 | from about 0.8 to about 3.0 | 350-650 |
| greater than about 1200 to about 2000 | from about 55 to about 80 | from about 24 to about 32 | from about 0.8 to about 3.0 | 500-1000 |
| greater than 2000 to about about 4000 | from about 55 to about 80 | from about 24 to about 32 | from about 0.8 to about 3.0 | 1000-2200 |

For larger sutures, i.e. sutures having an overall suture denier greater than about 300, the range of pick count, number of sheath yarns, denier of individual filaments and denier of the cabled core are related to each other as follows:

Table VI

| Braided Suture Construction Parameters | | | | |
|---|---|---|---|---|
| Overall Suture Denier | Pick Count | Number of Sheath Yarns | Denier of Individual Filaments | Denier of Cabled Core |
| greater than about 300 to about 500 | from about 50 to about 100 | from about 10 to about 20 | from about 0.2 to about 6.0 | 80-150 |
| greater than about 500 to about 800 | from about 50 to about 100 | from about 14 to about 20 | from about 0.2 to about 6.0 | 150-300 |
| greater than about 800 to about 1200 | from about 50 to about 100 | from about 16 to about 32 | from about 0.2 to about 6.0 | 250-700 |
| greater than about 1200 to about 2000 | from about 50 to about 100 | from about 20 to about 36 | from about 0.2 to about 6.0 | 400-1200 |
| greater than about 2000 to about 4000 | from about 50 to about 100 | from about 20 to about 36 | from about 0.2 to about 6.0 | 800-2400 |

In a more preferred braided suture construction of larger overall denier, the relationship of the structural elements of the suture for a given suture size is set forth in Table VII as follows:

Table VII

| More Preferred Braided Suture Construction Parameters | | | | |
|---|---|---|---|---|
| Overall Suture Denier | Pick Count | Number of Sheath Yarns | Denier of Individual Filaments | Denier of Cabled Core |
| greater than about 300 to about 500 | from about 55 to about 80 | from about 12 to about 14 | from about 0.8 to about 3.0 | 80-120 |
| greater than about 500 to about 800 | from about 55 to about 80 | from about 14 to about 18 | from about 0.8 to about 3.0 | 180-280 |
| greater than about 800 to about 1200 | from about 55 to about 80 | from about 20 to about 30 | from about 0.8 to about 3.0 | 350-650 |
| greater than about 1200 to about 2000 | from about 55 to about 80 | from about 24 to about 32 | from about 0.8 to about 3.0 | 500-1000 |
| greater than about 2000 about 4000 | from about 55 to about 80 | from about 24 to about 32 | from about 0.8 to about 3.0 | 1000-2200 |

As a result of their possessing a greater pick count and/or a greater number of sheath yarns for a suture of given overall denier and in some cases, a finer denier for the individual filaments making up a sheath yarn, the braided sutures of Tables IV-VII exhibit far fewer surface discontinuities, thereby providing sutures which are considerably smoother than braided sutures of known construction.

It can be advantageous to apply one or more coating compositions to the braided suture of this invention to improve such properties as surface lubricity, knot tie-down behavior, and so forth. A variety of suture coating compositions proposed for either or both purposes is known in the art, e.g., those disclosed in U.S. Patent No. 4,047,533.

It is also within the scope of this invention to impregnate the suture with, or otherwise apply thereto, one or more medico-surgically useful substances, e.g., those which accelerate or beneficially modify the healing process when the suture is applied to a wound or surgical site. So, for example, the braided suture herein can be provided with a therapeutic agent which will be deposited at the sutured site. The therapeutic agent can be chosen for its antimicrobial properties, capability for promoting wound repair and/or tissue growth or for specific indications such as thrombosis. Antimicrobial agents such as broad spectrum antibiotics (gentamycin sulphate, erythromycin or derivatized glycopeptides) which are slowly released into the tissue can be applied in this manner to aid in combating clinical and sub-clinical infections in a surgical or trauma wound site. To promote wound repair and/or tissue growth, one or several growth promoting factors can be introduced into the suture, e.g., human growth factors such as fibroblast growth factor, bone growth factor, epidermal growth factor, platelet-derived growth factor, macrophage-derived growth factor, alveolar-derived growth factor, monocyte-derived growth factor, magainin, carrier proteins, and so forth. Some therapeutic indications are: glycerol with tissue or kidney plasminogen activator to cause thrombosis, superoxide dismutase to scavenge tissue damaging free radicals, tumor necrosis factor for cancer therapy or colony stimulating factor and interferon, interleukin-2 or other lymphokine to enhance the immune system.

The following examples are illustrative of the braided suture of the invention. In all cases, the suture filaments were constructed from a 90-10 weight percent glycolide-L-lactide copolymer.

EXAMPLES 1-4

The cabled cores of Examples 1-4 were constructed in accordance with the parameters set forth in Table VIII as follows:

TABLE VIII

| Cabled Core Construction Characteristics | | | | | | | |
|---|---|---|---|---|---|---|---|
| Example | No. of Filaments In The Core | Denier of Individual Yarns In The Core | No. Of Yarns In Each Plied Yarn | First Twist In Turns Per Meter(applied to the plied yarn) | Second Twist In Turns Per Meter(applied to form the core) | Coefficient of Twist | Cabled Core Denier |
| 1[a] | 60 x 7 x 3 | 72 | 7 | 305 | 250 | 32 | 1512 |
| 2 | 35 x 7 x 3 | 42 | 7 | 534 | 409 | 40 | 882 |
| 3 | 22 x 7 x 3 | 26 | 7 | 583 | 447 | 35 | 554 |
| 4 | 28 x 3 x 3 | 33 | 3 | 823 | 630 | 37 | 297 |

[a] see formula (2) supra

Braided sutures were constructed around the cabled cores of Examples 1-4 in accordance with the parameters set forth in Table IX as follows:

TABLE IX

| Braided Suture Construction Characteristics | | | | | | | | |
|---|---|---|---|---|---|---|---|---|
| Core of Example | Suture Size | Overall Suture Denier | Pick Count | No. of Sheath Yarns | Denier of Individual Yarns | | % of Core | Braid Angle |
| | | | | | Sheath | Core | | |
| 1 | 1 | 2280 | 74 | 32 | 24 | 70 | 66 | 15.3 |
| 2 | 1/0 | 1550 | 80 | 28 | 24 | 40 | 57 | 17.4 |
| 3 | 2/0 | 1130 | 72 | 24 | 24 | 24 | 49 | 15.4 |
| 4 | 3/0 | 650 | 58 | 16 | 24 | 30 | 41 | 13.7 |

In no case did core popping occur with any of the foregoing sutures thus demonstrating the effectiveness of the cabled core in restraining any tendency of the core to penetrate the external sheath during handling of the suture.

EXAMPLES 5-11

The cabled cores of Examples 5-11 were constructed in accordance with the parameters set forth in Table X as follows:

TABLE X

| Cabled Core Construction Characteristics | | | | | | | |
|---|---|---|---|---|---|---|---|
| Example | No. of Filaments In The Core | Denier of Individual Yarns In Core | Denier Of Each Plied Yarn | Total Core Denier | Front Twist In Turns Per Meter Applied To Each Plied Yarn[b] | Back Twist In Turns Per Meter To Form Cable Core | Coefficient Of Back Twist |
| 5 | 69 x 7 x 3 | 110 | 770 | 2,310 | 250 | 278 | 44.5 |
| 6 | 48 x 7 x 3 | 77 | 540 | 1,617 | 320 | 324 | 43.5 |
| 7 | 27 x 7 x 3 | 43 | 300 | 900 | 590 | 505 | 50.5 |
| 8 | 17 x 7 x 3 | 27 | 190 | 570 | 640 | 576 | 45.8 |
| 9 | 54 x 1 x 3 | 86 | 86 | 258 | 830 | 854 | 45.7 |
| 10 | 23 x 1 x 3 | 37 | 37 | 110 | 1,000 | 1,035 | 36.3 |
| 11 | 17 x 1 x 3 | 27 | 27 | 80 | 1,200 | 1,200 | 36.0 |

[b] In Examples 9, 10 and 11, only unplied yarn was used

Braided sutures were constructed around the cabled cores of Examples 5-11 in accordance with the parameters set forth in Table XI as follows:

TABLE XI

| Braided Suture Construction Characteristics | | | | | | | | |
|---|---|---|---|---|---|---|---|---|
| Core of Example | Suture Size | Overall Suture Denier | Pick Count Picks Per Inch | No. of Sheath Yarns | Denier of | | % of Core | Braid Angle |
| | | | | | Sheath | Core | | |
| 5 | 2 | 3,190 | 79 | 32 | 870 | 2,320 | 73 | 18 |
| 6 | 1 | 2,380 | 79 | 32 | 770 | 1,610 | 68 | 18 |
| 7 | 0 | 1,580 | 83 | 28 | 670 | 910 | 58 | 17 |
| 8 | 2/0 | 1,150 | 88 | 24 | 580 | 570 | 50 | 17 |
| 9 | 3/0 | 690 | 63 | 16 | 430 | 260 | 38 | 16 |
| 10 | 4/0 | 440 | 79 | 12 | 330 | 110 | 25 | 16 |
| 11 | 5/0 | 300 | 55 | 8 | 220 | 80 | 27 | 15 |

COMPARATIVE EXAMPLES 12-16

The cabled cores of Comparative Examples 12-16 were constructed in accordance with the parameters set forth in Table XII as follows:

TABLE XII

| Compara-tive Example | No. of Fila-ments In The Core | Denier of Yarns In Each Ply[c] | No. Of Yarns In Each Ply[c] | First Twist In Turns Per Meter(applied to each plied yarn)[c] | Second Twist In Turns Per Meter (applied to the core) | Coefficient of Twist | Cabled Core Denier |
|---|---|---|---|---|---|---|---|
| Cabled Core Construction Parameters | | | | | | | |
| 12 | 110 x 3 x 3 | 132 | 3 | 193 | 120 | 14 | 1188 |
| 13 | 60 x 3 x 3 | 70 | 3 | 438 | 257 | 22 | 648 |
| 14 | 50 x 3 x 3 | 60 | 3 | 193 | 85 | 7 | 540 |
| 15 | 50 x 3 x 3 | 60 | 3 | 193 | 85 | 7 | 540 |
| 16 | 60 x 1 x 3 | 70 | 1 | 560 | 430 | 21 | 216 |

[c] In Comparative Example 16, only unplied yarn was used

Braided sutures were constructed around the cores of Comparative Examples 12-16 in accordance with the parameters set forth in Table XIII as follows:

TABLE XIII

| Core of Comparative Example | Suture Size | Overall Suture Denier | Pick Count | No. of Sheath Yarns | Denier of Individual Yarns | | % of Core | Braid Angle |
|---|---|---|---|---|---|---|---|---|
| Braided Suture Construction Characteristics | | | | | | | | |
| | | | | | Sheath | Core | | |
| 12 | 1 | 1960 | 79 | 32 | 24 | 132 | 60 | 15.7 |
| 13 | 1/0 | 1320 | 74 | 28 | 24 | 72 | 49 | 15.6 |
| 14 | 2/0 | 1120 | 86 | 24 | 24 | 60 | 48 | 20.0 |
| 15 | 2/0 | 1120 | 78 | 24 | 24 | 60 | 48 | 17.5 |
| 16 | 3/0 | 600 | 80 | 16 | 24 | 72 | 36 | 14.8 |

In every case core popping was observed. This appears to have been due to low coefficient of twist (below 24).

Claims

1. A surgical suture, comprising a braided sheath surrounding a core of twisted yarns, and the core is made of yarns twisted in a first direction, at least two of said twisted yarns being twisted together in a second direction to form a core **characterised in that** the core has a coefficient of twist $\alpha$ in said second direction of at least 24, wherein

$$\alpha = \frac{K}{100} \, (0.111D)^{\frac{1}{2}}$$

in which:

K = level of twist, expressed in turns per meter
D = denier of core

**EP 0 513 335 B1**

2. The braided suture of claim 1, wherein the coefficient of twist is at least 28.

3. The braided suture of claim 2, wherein the coefficient of twist is at least 32.

4. The braided suture of any one of the preceding claims, wherein the core yarns are made from individual filaments of a non-absorbable material.

5. The braided suture of claim 4 wherein the non-absorbable material is cotton, silk, polyamide or polyolefin.

6. The braided suture of claim 1, 2 or 3, wherein the core yarns are made from individual filaments of a bioabsorbable polymer.

7. The braided suture of claim 6 wherein the polymer is derived at least in part from one or more monomers selected from glycolic acid, glycolide, lactic acid and lactide.

8. The braided suture of any one of the preceding claims wherein the core yarns possess a twist having a coefficient of twist substantially equal to but opposite the coefficient of twist of the core in said second direction.

9. The braided suture of any one of the preceding claims, wherein the core is constructed from a plurality of individual yarns possessing a first twist of from 100 to 1500 turns per meter, the core possessing a second, opposite twist of from 100 to 1200 turns per meter.

10. The braided suture of claim 9 wherein the first twist is of from 200 to 1200 turns per meter, and the second, opposite twist is of from 300 to 1100 turns per meter.

11. The braided suture of any one of the preceding claims, wherein said twisted yarns are plied prior to said second twist.

12. The braided suture of any one of the preceding claims, surface coated with a composition enhancing one or more functional properties of the suture.

13. The braided suture of any one of the preceding claims containing at least one medico-surgically useful substance.

14. A braided suture as claimed in any one of the preceding claims, wherein the overall suture denier and the denier of the core are related as follows:

| OVERALL SUTURE DENIER | DENIER OF CORE |
|---|---|
| greater than 125 to 200 | 20-80 |
| greater than 200 to 300 | 30-100 |
| greater than 300 to 500 | 80-150 |
| greater than 500 to 800 | 150-300 |
| greater than 800 to 1200 | 250-700 |
| greater than 1200 to 2000 | 400-1200 |
| greater than 2000 to 4000 | 800-2400 |

15. A braided suture as claimed in claim 14, wherein for a given range of overall suture denier, the range of pick count, number of sheath yarns, denier of individual filaments contained in a sheath yarn and denier of the cabled core are related to each other as follows:

| Overall Suture Denier | Pick Count | Number of Sheath Yarns | Denier of Individual Filaments | Denier of Cabled Core |
|---|---|---|---|---|
| greater than 125 to 200 | from 50 to 100 | from 4 to 16 | from 0.2 to 1.8 | 20-80 |
| greater than 200 to 300 | from 50 to 100 | from 4 to 16 | from 0.2 to 1.8 | 30-100 |
| greater than 300 to 500 | from 50 to 100 | from 10 to 20 | from 0.2 to 6.0 | 80-150 |
| greater than 500 to 800 | from 50 to 100 | from 14 to 20 | from 0.2 to 6.0 | 150-300 |
| greater than 800 to 1200 | from 50 to 100 | from 16 to 32 | from 0.2 to 6.0 | 250-700 |
| greater than 1200 to 2000 | from 50 to 100 | from 20 to 36 | from 0.2 to 6.0 | 400-1200 |
| greater than 2000 to 4000 | from 50 to 100 | from 20 to 36 | from 0.2 to 6.0 | 800-2400 |

16. A braided suture according to any one of claims 1 to 13, wherein the overall suture denier and the denier of the core are related as follows:

| OVERALL SUTURE DENIER | DENIER OF CORE |
|---|---|
| greater than 125 to 200 | 25-50 |
| greater than 200 to 300 | 50-80 |
| greater than 300 to 500 | 80-120 |
| greater than 500 to 800 | 180-280 |
| greater than 800 to 1200 | 350-650 |
| greater than 1200 to 2000 | 500-1000 |
| greater than 2000 to 4000 | 1000-2200 |

17. The braided suture of claim 16, wherein for a given overall suture denier, the range of pick count, number of sheath yarns, denier of individual filaments comprising a sheath yarn and denier of the cabled core are related to each

other as follows:

| Overall Suture Denier | Pick Count | Number of Sheath Yarns | Denier of Individual Filaments | Denier of Cabled Core |
|---|---|---|---|---|
| greater than 125 to 200 | from 55 to 80 | from 6 to 14 | from 0.8 to 1.4 | 25-50 |
| greater than 200 to 300 | from 55 to 80 | from 6 to 14 | from 0.8 to 1.4 | 50-80 |
| greater than 300 to 500 | from 55 to 80 | from 12 to 14 | from 0.8 to 3.0 | 80-120 |
| greater than 500 to 800 | from 55 to 80 | from 14 to 18 | from 0.8 to 3.0 | 80-280 |
| greater than 800 to 1200 | from 55 to 80 | from 20 to 30 | from 0.8 to 3.0 | 350-650 |
| greater than 1200 to 2000 | from 55 to 80 | from 24 to 32 | from 0.8 to 3.0 | 500-1000 |
| greater than 2000 to 4000 | from 55 to 80 | from 24 to 32 | from 0.8 to 3.0 | 1000-2200 |

**18.** A braided suture as claimed in any one of claims 1 to 13, wherein for a given range of overall suture denier, the range of pick count, number of sheath yarns, denier of individual filaments comprising a sheath yarn and denier of the cabled core are related to each other as follows:

| Overall Suture Denier | Pick Count | Number of Sheath Yarns | Denier of Individual Filaments | Denier of Cabled Core |
|---|---|---|---|---|
| greater than 300 to 500 | from 50 to 100 | from 10 to 20 | from 0.2 to 6.0 | 80-150 |

| Overall Suture Denier | Pick Count | Number of Sheath Yarns | Denier of Individual Filaments | Denier of Cabled Core |
|---|---|---|---|---|
| greater than 500 to 800 | from 50 to 100 | from 14 to 20 | from 0.2 to 6.0 | 150-300 |
| greater than 800 to 1200 | from 50 to 100 | from 16 to 32 | from 0.2 to 6.0 | 250-700 |
| greater than 1200 to 2000 | from 50 to 100 | from 20 to 36 | from 0.2 to 6.0 | 400-1200 |
| greater than 2000 to 4000 | from 50 to 100 | from 20 to 36 | from 0.2 to 6.0 | 800-2400 |

**19.** The braided suture of claim 18, wherein for a given range of overall suture denier, the range of pick count, number of sheath yarns, denier of individual filaments comprising a sheath yarn and denier of the cabled core are related to each other as follows:

| Overall Suture Denier | Pick Count | Number of Sheath Yarns | Denier of Individual Filaments | Denier of Cabled Core |
|---|---|---|---|---|
| greater than 300 to 500 | from 55 to 80 | from 12 to 14 | from 0.8 to 3.0 | 80-120 |
| greater than 500 to 800 | from 55 to 80 | from 14 to 18 | from 0.8 to 3.0 | 180-280 |
| greater than 800 to 1200 | from 55 to 80 | from 20 to 30 | from 0.8 to 3.0 | 350-650 |
| greater than 1200 to 2000 | from 55 to 80 | from 24 to 34 | from 0.8 to 3.0 | 500-1000 |
| greater than 2000 to 4000 | from 55 to 80 | from 24 to 34 | from 0.8 to 3.0 | 1000-2200 |

**20.** A method of making a core for a braided suture comprising:

(i) twisting yarns in a first direction; and
(ii) twisting at least two of said twisted yarns together in a second direction opposite said first direction to form the core **characterised by** forming the core with a coefficient of twist $\alpha$ of said core in said second direction of at least 24, wherein

$$\alpha = \frac{K}{100} (0.111D)^{1/2}$$

in which:

K = twist level in turns per meter; and
D = denier of said core.

21. The method of claim 20 wherein said step of twisting yarns comprises twisting said yarns to possess a coefficient of twist in said first direction substantially equal to the coefficient of twist in said second direction.

22. The method of claim 20 or 21 wherein the coefficient of twist of the core is at least 28.

23. The method of claim 22 wherein the coefficient of twist of the core is at least 32.

24. The method of any one of claims 20 to 23, wherein said step of twisting yarns comprises twisting said yarns in said first direction from about 100 to 1500 turns per meter.

25. The method of claim 24 wherein said steps of twisting yarns comprises twisting said yarns in said first direction from about 200 to about 1200 turns per meter.

26. The method of any one of claims 20 to 25, wherein said step of twisting in a second direction comprises twisting said twisted yarns from about 100 to 1200 turns per meter.

27. The method of claim 26, wherein said step of twisting in a second direction comprises twisting said twisted yarns from about 300 to about 1100 turns per meter.

28. The method of any one of claims 20 to 27, further comprising the step of braiding sheath yarns around said cabled core to form a suture.

29. A method according to any one of claims 20 to 28, including the step of plying at least two of the yarns together before applying to said plied yarns said step of twisting in a first direction.

**Patentansprüche**

1. Chirurgischer Faden mit einem geflochtenen Mantel, der einen Kern verdrillter Garne umgibt, wobei der Kern aus Garnen hergestellt ist, die in einer ersten Richtung verdrillt sind, wobei wenigstens zwei der verdrillten Garne miteinander in einer zweiten Richtung verdrillt sind, um einen Kern zu bilden, dadurch gekennzeichnet, daß der Kern einen Verdrillungskoeffizienten $\alpha$ in der zweiten Richtung von wenigstens 24 aufweist, wobei

$$\alpha = \frac{K}{100} (0{,}111D)^{1/2}$$

wobei folgendes gilt:

K = Verdrillungsstärke, ausgedrückt in Umdrehungen pro Meter,
D = Denier des Kerns

2. Geflochtener Faden nach Anspruch 1, wobei der Verdrillungskoeffizient wenigstens 28 ist.

3. Geflochtener Faden nach Anspruch 2, wobei der Verdrillungskoeffizient wenigstens 32 ist.

4. Geflochtener Faden nach einem der vorangehenden Ansprüche, wobei die Kerngarne aus einzelnen Fasern aus einem nicht absorbierbaren Material hergestellt sind.

5. Geflochtener Faden nach Anspruch 4, wobei das nichtabsorbierbare Material Baumwolle, Seide, Polyamid oder Polyolefin ist.

6. Geflochtener Faden nach Anspruch 1, 2 oder 3, wobei die Kerngarne aus einzelnen Fasern aus biologisch absorbierbarem Polymer hergestellt sind.

**7.** Geflochtener Faden nach Anspruch 6, wobei das Polymer wenigstens teilweise aus einem oder mehreren Monomeren abgeleitet ist, die aus Glycolsäure, Glycolid, Lacticsäure und Lactid ausgewählt sind.

**8.** Geflochtener Faden nach einem der vorangehenden Ansprüche, wobei die Kerngarne eine Verdrillung besitzen, die einen Verdrillungskoeffizienten hat, der im wesentlichen gleich aber entgegengesetzt zu dem Verdrillungskoeffizienten des Kerns in der zweiten Richtung ist.

**9.** Geflochtener Faden nach einem der vorangehenden Ansprüche, wobei der Kern aus einer Vielzahl einzelner Garne hergestellt ist, die eine erste Verdrillung von 100 bis 1500 Umdrehungen pro Metern besitzen, wobei der Kern eine zweite entgegengesetzte Verdrillung von 100 bis 1200 Umdrehungen pro Metern besitzt.

**10.** Geflochtener Faden nach Anspruch 9, wobei die erste Verdrillung von 200 bis 1200 Umdrehungen pro Metern erfolgt, und die zweite entgegengesetzte Verdrillung von 300 bis 1100 Umdrehungen pro Metern erfolgt.

**11.** Geflochtener Faden nach einem der vorangehenden Ansprüche, wobei die verdrillten Garne vor der zweiten Verdrillung in eine Richtung gelegt sind.

**12.** Geflochtener Faden nach einem der vorangehenden Ansprüche, wobei eine Oberfläche, die mit einer Zusammensetzung überzogen ist, eine oder mehrere funktionelle Eigenschaften des Fadens verstärkt.

**13.** Geflochtener Faden nach einem der vorangehenden Ansprüche, der wenigstens eine medizinisch chirurgisch nützliche Substanz enthält.

**14.** Geflochtener Faden nach einem der vorangehenden Ansprüche, wobei der Gesamtfaden-Denier und der Denier des Kerns folgendermaßen aufeinander bezogen sind:

| GESAMTFADEN-DENIER | DENIER DES KERNS |
|---|---|
| größer als 125 bis 200 | 20-80 |
| größer als 200 bis 300 | 30-100 |
| größer als 300 bis 500 | 80-150 |
| größer als 500 bis 800 | 150-300 |
| größer als 800 bis 1200 | 250-700 |
| größer als 1200 bis 2000 | 400-1200 |
| größer als 2000 bis 4000 | 800-2400 |

**15.** Geflochtener Faden nach Anspruch 14, wobei für einen gegebenen Bereich des Gesamtfaden-Deniers der Bereich einer Schußzahl, eine Anzahl von Mantelgarnen, der Denier der einzelnen Fasern, die in einem Mantelgarn enthalten sind, und der Denier des gewundenen Kerns wie folgt aufeinander bezogen sind:

| Gesamtfaden-Denier | Schußzahl | Anzahl von Mantelgarnen | Denier einzelner Fasern | Denier des gewun-denen Kerns |
|---|---|---|---|---|
| größer als 125 bis 200 | von 50 bis 100 | von 4 bis 16 | von 0,2 bis 1,8 | 20-80 |
| größer als 200 bis 300 | von 50 bis 100 | von 4 bis 16 | von 0,2 bis 1,8 | 30-100 |
| größer als 300 bis 500 | von 50 bis 100 | von 10 bis 20 | von 0,2 bis 6,0 | 80-150 |
| größer als 500 bis 800 | von 50 bis 100 | von 14 bis 20 | von 0,2 bis 6,0 | 150-300 |
| größer als 800 bis 1200 | von 50 bis 100 | von 16 bis 32 | von 0,2 bis 6,0 | 250-700 |
| größer als 1200 bis 2000 | von 50 bis 100 | von 20 bis 36 | von 0,2 bis 6,0 | 400-1200 |
| größer als 2000 bis 4000 | von 50 bis 100 | von 20 bis 36 | von 0,2 bis 6,0 | 800-2400 |

16. Geflochtener Faden nach einem der Ansprüche 1 bis 13, wobei der Gesamtfaden-Denier und der Denier des Kerns wie folgt aufeinander bezogen sind:

| GESAMTFADEN-DENIER | DENIER DES KERNS |
|---|---|
| größer als 125 bis 200 | 25-50 |
| größer als 200 bis 300 | 50-80 |
| größer als 300 bis 500 | 80-120 |
| größer als 500 bis 800 | 180-280 |
| größer als 800 bis 1200 | 350-650 |
| größer als 1200 bis 2000 | 500-1000 |
| größer als 2000 bis 4000 | 1000-2000 |

17. Geflochtener Faden nach Anspruch 16, wobei für einen gegebenen Gesamtfaden-Denier der Bereich der Schußzahl, die Anzahl von Mantelgarnen, der Denier der einzelnen Fasern mit einem Mantelgarn und der Denier des gewundenen Kerns wie folgt aufeinander bezogen sind:

| Gesamtfaden-Denier | Schußzahl | Anzahl von Mantelgarnen | Denier einzelner Fasern | Denier des gewundenen Kerns |
|---|---|---|---|---|
| größer als 125 bis 200 | von 55 bis 80 | von 6 bis 14 | von 0,8 bis 1,4 | 25-50 |
| größer als 200 bis 300 | von 55 bis 80 | von 6 bis 14 | von 0,8 bis 1,4 | 50-80 |
| größer als 300 bis 500 | von 55 bis 80 | von 12 bis 14 | von 0,8 bis 3,0 | 80-120 |
| größer als 500 bis 800 | von 55 bis 80 | von 14 bis 18 | von 0,8 bis 3,0 | 80-280 |
| größer als 800 bis 1200 | von 55 bis 80 | von 20 bis 30 | von 0,8 bis 3,0 | 350-650 |
| größer als 1200 bis 2000 | von 55 bis 80 | von 24 bis 32 | von 0,8 bis 3,0 | 500-1000 |
| größer als 2000 bis 4000 | von 55 bis 80 | von 24 bis 32 | von 0,8 bis 3,0 | 1000-2000 |

18. Geflochtener Faden nach einem der Ansprüche 1 bis 13, wobei für einen gegebenen Bereich des Gesamtfaden-Deniers der Bereich der Schußzahl, die Anzahl der Mantelgarne, der Denier der einzelnen Fasern mit einem Mantelgarn und der Denier des gewundenen Kerns wie folgt aufeinander bezogen sind:

| Gesamtfaden-Denier | Schußzahl | Anzahl von Mantelgarnen | Denier einzelner Fasern | Denier des gewundenen Kerns |
|---|---|---|---|---|
| größer als 300 bis 500 | von 50 bis 100 | von 10 bis 20 | von 0,2 bis 6,0 | 80-150 |
| größer als 500 bis 800 | von 50 bis 100 | von 14 bis 20 | von 0,2 bis 6,0 | 150-300 |
| größer als 800 bis 1200 | von 50 bis 100 | von 16 bis 32 | von 0,2 bis 6,0 | 250-700 |
| größer als 1200 bis 2000 | von 50 bis 100 | von 20 bis 36 | von 0,2 bis 6,0 | 400-1200 |
| größer als 2000 bis 4000 | von 50 bis 100 | von 20 bis 36 | von 0,2 bis 6,0 | 800-2400 |

**19.** Geflochtener Faden nach Anspruch 18, wobei für einen gegebenen Bereich des Gesamtfaden-Deniers der Bereich der Schußzahl, die Anzahl der Mantelgarne, der Denier der einzelnen Fasern mit einem Mantelgarn und der Denier des gewundenen Kerns wie folgt aufeinander bezogen sind:

| Gesamtfaden-Denier | Schußzahl | Anzahl von Mantelgarnen | Denier einzelner Fasern | Denier des gewundenen Kerns |
|---|---|---|---|---|
| größer als 300 bis 500 | von 55 bis 80 | von 12 bis 14 | von 0,8 bis 3,0 | 80-120 |
| größer als 500 bis 800 | von 55 bis 80 | von 14 bis 18 | von 0,8 bis 3,0 | 180-280 |
| größer als 800 bis 1200 | von 55 bis 80 | von 20 bis 30 | von 0,8 bis 3,0 | 350-650 |
| größer als 1200 bis 2000 | von 55 bis 80 | von 24 bis 34 | von 0,8 bis 3,0 | 500-1000 |
| größer als 2000 bis 4000 | von 55 bis 80 | von 24 bis 34 | von 0,8 bis 3,0 | 1000-2000 |

**20.** Verfahren zum Herstellen eines Kerns für einen geflochtenen Faden, das folgendes aufweist:

(i) Verdrillen von Garnen in einer ersten Richtung; und
(ii) Verdrillen wenigstens zweier der verdrillten Garne miteinander in einer zweiten Richtung, die entgegengesetzt zu der ersten Richtung ist, um den Kern zu bilden, gekennzeichnet durch Bilden des Kerns mit einem Koeffizienten einer Verdrillung $\alpha$ des Kerns in der zweiten Richtung von wenigstens 24, wobei

$$\alpha = \frac{K}{100} \, (0{,}111D)^{1/2}$$

wobei folgendes gilt:

K = Verdrillungspegel in Umdrehungen pro Meter;
D = Denier des Kerns.

**21.** Verfahren nach Anspruch 20, wobei der Schritt eines Verdrillens von Garnen ein Verdrillen der Garne aufweist, damit sie einen Koeffizienten der Verdrillung in der ersten Richtung besitzen, der in wesentlichen gleich dem Koeffizienten der Verdrillung in der zweiten Richtung ist.

**22.** Verfahren nach Anspruch 20 oder 21, wobei der Koeffizient der Verdrillung des Kerns wenigstens 28 ist.

**23.** Verfahren nach Anspruch 22, wobei der Koeffizient der Verdrillung des Kerns wenigstens 32 ist.

**24.** Verfahren nach einem der Ansprüche 20 bis 23, wobei der Schritt zum Verdrillen von Garnen ein Verdrillen der Garne in der ersten Richtung von etwa 100 bis 1500 Umdrehungen pro Meter aufweist.

**25.** Verfahren nach Anspruch 24, wobei die Schritte zum Verdrillen von Garnen ein Verdrillen der Garne in der ersten Richtung von etwa 200 bis etwa 1200 Umdrehungen pro Meter aufweist.

**26.** Verfahren nach einem der Ansprüche 20 bis 25, wobei der Schritt zum Verdrillen in einer zweiten Richtung ein Verdrillen der verdrillten Garne von etwa 100 bis 1200 Umdrehungen pro Meter aufweist.

**27.** Verfahren nach Anspruch 26, wobei der Schritt zum Verdrillen in einer zweiten Richtung ein Verdrillen der verdrillten Garne von etwa 300 bis etwa 1100 Umdrehungen pro Meter aufweist.

**28.** Verfahren nach einem der Ansprüche 20 bis 27, das weiterhin den Schritt zum Flechten von Mantelgarnen um den gewundenen Kern zum Bilden eines Fadens aufweist.

**29.** Verfahren nach einem der Ansprüche 20 bis 28, das den Schritt zum Legen wenigstens zweier der Garne miteinander in eine Reihe vor einem Anwenden des Schritts zum Verdrillen in einer ersten Richtung auf die in eine Reihe gelegten Garne.

## Revendications

**1.** Suture chirurgicale, comprenant une enveloppe tressée entourant une âme de fils torsadés, et l'âme est faite de fils torsadés dans une première direction, au moins deux desdits fils torsadés étant torsadés ensemble dans une seconde direction pour former une âme, caractérisée en ce que l'âme a un coefficient de torsion $\alpha$ dans ladite seconde direction d'au moins 24, où

$$\alpha = \frac{K}{100} \, (O{,}111D)^{1/2}$$

où :

K = niveau de torsion, exprimé en tours par mètre
D = denier de l'âme.

**2.** Suture tressée selon la revendication 1, où le coefficient de torsion est d'au moins 28.

**3.** Suture tressée selon la revendication 2, où le coefficient de torsion vaut au moins 32.

4. Suture tressée selon l'une quelconque des revendications précédentes, où les fils de l'âme sont faits à partir de filaments individuels d'un matériau non absorbable.

5. Suture tressée selon la revendication 4, où le matériau non absorbable est du coton, de la soie, un polyamide ou une polyoléfine.

6. Suture tressée selon la revendication 1, 2 ou 3, où les fils de l'âme sont faits à partir de filaments individuels d'un polymère bioabsorbable.

7. Suture tressée selon la revendication 6 où le polymère est dérivé au moins en partie d'un ou plus monomères choisis parmi l'acide glycolique, le glycolide, l'acide lactique et le lactide.

8. Suture tressée selon l'une quelconque des revendications précédentes dans laquelle les fils de l'âme possèdent une torsade ayant un coefficient de torsion substantiellement égal à mais opposé au coefficient de torsion de l'âme dans ladite seconde direction.

9. Suture tressée selon l'une quelconque des revendications précédentes, où l'âme est construite à partir d'une pluralité de fils individuels possédant une première torsade de 100 à 1500 tours par mètre, l'âme possédant une seconde torsade, opposée, de 100 à 1200 tours par mètre.

10. Suture tressée selon la revendication 9 où la première torsade est de 200 à 1200 tours par mètre, et la seconde torsade, opposée, est de 300 à 1500 tours par mètre.

11. Suture tressée selon l'une quelconque des revendications précédentes, où lesdits fils torsadés sont pliés avant ladite seconde torsade.

12. Suture tressée selon l'une quelconque des revendications précédentes, revêtue en surface d'une composition augmentant une ou plus propriétés fonctionnelles de la suture.

13. Suture tressée selon l'une quelconque des revendications précédentes contenant au moins une substance utile médico-chirurgicalement.

14. Suture tressée selon l'une quelconque des revendications précédentes, où le denier de suture globale et le denier de l'âme sont reliés comme suit :

| DENIER DE SUTURE GLOBAL | DENIER DE L'ÂME |
|---|---|
| supérieur à 125 à 200 | 20-80 |
| supérieur à 200 à 300 | 30-100 |
| supérieur à 300 à 500 | 80-150 |
| supérieur à 500 à 800 | 150-300 |
| supérieur à 800 à 1200 | 250-700 |
| supérieur à 1200 à 2000 | 400-1200 |
| supérieur à 2000 à 4000 | 800-2400 |

15. Suture tressée selon la revendication 14, où pour un intervalle donné de deniers de suture globaux, l'intervalle de comptage de pics, le nombre de fils d'enveloppe, le denier des filaments individuels contenus dans un fil d'enveloppe et le denier de l'âme câblée sont reliés l'un à l'autre comme suit :

| Denier de suture global | Comptage des pics | Nombre de fils d'enveloppe | Denier des filaments individuels | Denier de l'âme câblée |
|---|---|---|---|---|
| Supérieur à 125 à 200 | de 50 à 100 | de 4 à 16 | de 0,2 à 1,8 | 20-80 |
| supérieur à 200 à 300 | de 50 à 100 | de 4 à 16 | de 0,2 à 1,8 | 30-100 |
| supérieur à 300 à 500 | de 50 à 100 | de 10 à 20 | de 0,2 à 6,0 | 80-150 |
| supérieur à 500 à 800 | de 50 à 100 | de 14 à 20 | de 0,2 à 6,0 | 150-300 |
| supérieur à 800 à 1200 | de 50 à 100 | de 16 à 32 | de 0,2 à 6,0 | 250-700 |
| supérieur à 1200 à 2000 | de 50 à 100 | de 20 à 36 | de 0,2 à 6,0 | 400-1200 |
| supérieur à 2000 à 4000 | de 50 à 100 | de 20 à 36 | de 0,2 à 6,0 | 800-2400 |

16. Suture tressée selon l'une quelconque des revendications 1 à 13, où le denier de suture global et le denier de l'âme sont reliés comme suit :

| DENIER DE SUTURE GLOBAL | DENIER DE L'ÂME |
|---|---|
| supérieur à 125 à 200 | 25-50 |
| supérieur à 200 à 300 | 50-80 |
| supérieur à 300 à 500 | 80-120 |
| supérieur à 500 à 800 | 180-280 |
| supérieur à 800 à 1200 | 350-650 |
| supérieur à 1200 à 2000 | 500-1000 |
| supérieur à 2000 à 4000 | 1000-2200 |

**17.** Suture tressée selon la revendication 16, où pour un denier de suture global donné, l'intervalle de comptage de pics, le nombre des fils d'enveloppe, le denier des filaments individuels comprenant un fil d'enveloppe et le denier de l'âme câblée sont reliés l'un à l'autre comme suit

| Denier de suture global | Comptage des pics | Nombre de fils d'enveloppe | Denier des filaments individuels | Denier de l'âme câblée |
|---|---|---|---|---|
| Supérieur à 125 à 200 | de 55 à 80 | de 6 à 14 | de 0,8 à 1,4 | 25-50 |
| supérieur à 200 à 300 | de 55 à 80 | de 6 à 14 | de 0,8 à 1,4 | 50-80 |
| supérieur à 300 à 500 | de 55 à 80 | de 12 à 14 | de 0,8 à 3,0 | 80-120 |
| supérieur à 500 à 800 | de 55 à 80 | de 14 à 18 | de 0,8 à 3,0 | 80-280 |
| supérieur à 800 à 1200 | de 55 à 80 | de 20 à 30 | de 0,8 à 3,0 | 350-650 |
| supérieur à 1200 à 2000 | de 55 à 80 | de 24 à 32 | de 0,8 à 3,0 | 500-1000 |
| supérieur à 2000 à 4000 | de 55 à 80 | de 24 à 32 | de 0,8 à 3,0 | 1000-2200 |

**18.** Suture tressée selon l'une quelconque des revendications 1 à 13, où pour un intervalle donné de deniers de suture globaux, l'intervalle de comptage des pics, le nombre des filaments d'enveloppe, le denier des filaments individuels comprenant un fil d'enveloppe et le denier de l'âme câblée sont reliés l'un à l'autre comme suit :

| Denier de suture global | Comptage des pics | Nombre de fils d'enveloppe | Denier des filaments individuels | Denier de l'âme de câble |
|---|---|---|---|---|
| supérieur à 300 à 500 | de 50 à 100 | de 10 à 20 | de 0,2 à 6,0 | 80-150 |
| supérieur à 500 à 800 | de 50 à 100 | de 14 à 18 | de 0,2 à 6,0 | 150-300 |
| supérieur à 800 à 1200 | de 50 à 100 | de 16 à 32 | de 0,2 à 6,0 | 250-700 |
| supérieur à 1200 à 2000 | de 50 à 100 | de 20 à 36 | de 0,2 à 6,0 | 400-1200 |
| supérieur à 2000 à 4000 | de 50 à 100 | de 20 à 36 | de 0,2 à 6,0 | 800-2400 |

19. Suture tressée selon la revendication 18, où pour un intervalle donné de denier de suture global, l'intervalle de comptage des pics, le nombre de fils d'enveloppe, le denier des filaments individuels comprenant un fil d'enveloppe et le denier de l'âme câblée sont reliés l'un à l'autre comme suit :

| Denier de suture global | Comptage des pics | Nombre de fils d'enveloppe | Denier des filaments individuels | Denier de l'âme câblée |
|---|---|---|---|---|
| supérieur à 300 à 500 | de 55 à 80 | de 12 à 14 | e 0,8 à 3,0 | 80-120 |
| supérieur à 500 à 800 | de 55 à 80 | de 14 à 18 | de 0,8 à 3,0 | 180-280 |
| supérieur à 800 à 1200 | de 55 à 80 | de 20 à 30 | de 0,8 à 3,0 | 350-650 |
| supérieur à 1200 à 2000 | de 55 à 80 | de 24 à 34 | de 0,8 à 3,0 | 500-1000 |
| supérieur à 2000 à 4000 | de 55 à 80 | de 24 à 34 | de 0,8 à 3,0 | 1000-2200 |

20. Méthode de fabrication d'une âme pour une suture tressée comprenant :

(i) le torsadage de fils dans une première direction; et
(ii) le torsadage d'au moins deux desdits fils torsadés ensemble dans une seconde direction opposée à ladite première direction pour former l'âme caractérisée par la formation de l'âme avec un coefficient de torsion $\alpha$ de ladite âme dans ladite seconde direction d'au moins 24, où

$$\alpha = \frac{K}{100} (O,111D)^{\frac{1}{2}}$$

où :

K = niveau de torsion en tours par mètre;
   et
D = denier de ladite âme.

21. Méthode selon la revendication 20 où ladite étape de torsadage des fils comprend le torsadage desdits fils pour posséder un coefficient de torsion dans ladite première direction substantiellement égal au coefficient de torsion dans ladite seconde direction.

22. Méthode selon la revendication 20 ou 21 où le coefficient de torsion de l'âme vaut au moins 28.

23. Méthode selon la revendication 22 où le coefficient de torsion de l'âme vaut au moins 32.

24. Méthode selon l'une quelconque des revendications 20 à 23, où ladite étape de torsadage des fils comprend le torsadage des fils dans ladite première direction d'environ 100 à 1500 tours par mètre.

25. Méthode selon la revendication 24 où lesdites étapes de torsadage des fils comprend le torsadage desdits fils dans ladite première direction d'environ 200 à environ 1200 tours par mètre.

26. Méthode selon l'une quelconque des revendications 20 à 25, où ladite étape de torsadage dans une seconde direction comprend le torsadage desdits fils torsadés d'environ 100 à 1200 tours par mètre.

27. Méthode selon la revendication 26, où ladite étape de torsadage dans une seconde direction comprend le torsadage desdits fils torsadés d'environ 300 à environ 1100 tours par mètre.

28. Méthode selon l'une quelconque des revendications 20 à 27, comprenant de plus l'étape de tressage des fils d'enveloppe autour de ladite âme câblée pour former une suture.

29. Méthode selon l'une quelconque des revendications 20 à 28, incluant l'étape de pliage d'au moins deux des fils ensemble avant application auxdits fils pliés de ladite étape de torsadage dans une première direction.